(19) Europäisches Patentamt
European Patent Office
Office européen des brevets

(11) Publication number : 0 510 839 A1

# (12) EUROPEAN PATENT APPLICATION

(21) Application number : 92303173.6

(51) Int. Cl.$^5$ : C08F 2/50, G03F 7/029

(22) Date of filing : 09.04.92

(30) Priority : 13.04.91 GB 9108132

(43) Date of publication of application : 28.10.92 Bulletin 92/44

(84) Designated Contracting States : AT BE CH DE FR GB IT LI NL

(71) Applicant : CIBA-GEIGY AG
Klybeckstrasse 141
CH-4002 Basel (CH)

(72) Inventor : Taylor, David Alan
57 Oxford Road
Cambridge, CB4 3PH (GB)
Inventor : Irving, Edward
Applecroft, Dark Lane
Higher Whitley, Warrington (GB)
Inventor : Lunn, Robert James
26 Hillcrest
Bar Hill, Cambridge (GB)

(74) Representative : Sharman, Thomas et al
CIBA-GEIGY PLC. Patent Department, Central Research, Hulley Road
Macclesfield, Cheshire SK10 2NX (GB)

## (54) Sulphoxonium salts.

(57) The invention provides an aryl sulphoxonium salt which either contains a polymerizable group in the molecule or is part of a polymeric compound.
The compounds are useful as cationic photoinitiators.

EP 0 510 839 A1

The present invention relates to novel sulphoxonium salts.

Many sulphoxonium salts are known, as are their uses as initiators for the photopolymerisation of cationically polymerizable materials such as epoxide resins. We have now developed new sulphoxonium salts which have higher compatibility with epoxide resins and liberate less volatile or leachable material into cured films which in many cases also means that less odour is produced.

Accordingly, the present invention provides an aryl sulphoxonium salt which either contains a polymerizable group in the molecule or is part of a polymeric compound.

The sulphoxonium salt of the invention may be a triaryl sulphoxonium salt where one of the aryl groups is pendant on a polymeric chain or is substituted by a polymerizable group or where the sulphoxonium group is in the polymer backbone.

Suitable polymerizable groups are those containing ethylenic unsaturation e.g. vinyl, allyl, acrylic or methacrylic groups, or a 1,2-epoxide group. The polymerizable group may be linked to the aryl group by a chain of from 1 to 4 carbon atoms optionally interrupted by 1 or 2 oxygen atoms.

When the aryl group is attached to a polymeric chain it may be attached directly to the chain or to the pendant groups on the chain e.g. via phenyl or phenol groups or via an oxygen atom.

Examples of compounds containing a polymerizable group are those of the general formula (I)

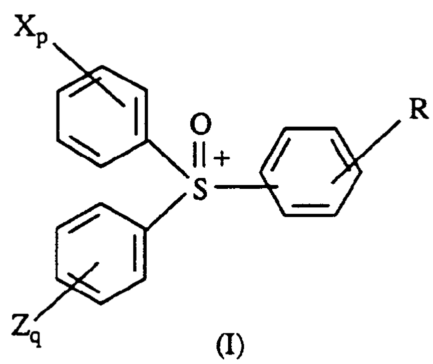

(I)

where R contains a (meth)acrylate, a vinyl ether, allyl ether or an epoxy group, X and Z, which may be the same or different, represent monovalent substituents such as halogen or alkyl groups, and p and q, which may be the same or different are either zero or integers below 6. For example R may be $CH_2$=CHCOO-, $CH_2$=C($CH_3$)COO-, $CH_2$=CHCOOCH$_2$CH(OH)CH$_2$O-, $CH_2$=C($CH_3$)COOCH$_2$CH(OH)CH$_2$0-, $CH_2$=CH-CH$_2$O-, or

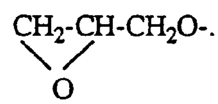

Examples of compounds in which the aryl group is attached to a polymeric chain include compounds where the polymer is one containing phenyl groups or is an epoxy resin. Suitable polymers containing phenyl groups include poly($\alpha$-methylstyrene), poly(4-vinylphenol), and phenol novolaks.

When the sulphoxonium salt is part of a polymeric compound it may be present on the chain at from 25 to 75% of the possible sites, or the sulphoxonium salt may be present as the terminating group of an oligomer.

The anion of the sulphoxonium salt may be a simple anion such as halide, e.g. chloride or bromide, trifluoromethanesulphonate, or preferably an ion of the formula (II)

$$MY^{-}_{m} \quad (II)$$

where M is an atom of a metal or metalloid, Y is a halogen atom such as fluorine or chlorine, and m is 4,5 or 6 and is one more than the valency of M. M may be boron, bismuth, antimony, arsenic or phosphorus. Thus the ion may be $BiCl_6^-$, $BF_4^-$, $PF_6^-$, $SbF_6^-$ or $AsF_6^-$.

Compounds of the invention containing a polymerizable group may be produced either by reacting an aryl sulphoxonium salt containing a reactive group, with a compound which will introduce the desired polymerizable group, or by reacting an aryl sulphonium salt containing a reactive group with a compound which will introduce the desired polymerisable group and then oxidising the sulphonium moieties to sulphoxonium moieties.

Suitable sulphoxonium compounds include those having a phenolic hydroxy group or a halogen atom. Compounds which introduce the polymerizable group include (meth)acryloyl halides, glycidyl (meth) acrylate,

allyl alcohol, vinyl alcohol, glycidol, allyl halides and epoxy resins, which may have been advanced.

Compounds of the invention in which the sulphoxonium group is part of a polymer may be prepared either by reacting a polymer containing reactive groups such as hydroxy or halogen with a sulphonium or sulphoxonium salt containing a group reactive with the reactive group on the polymer and if necessary converting the sulphonium salt into the corresponding sulphoxonium salt by methods known per se, or by reacting an aromatic polymer with a diaryl sulphoxide to produce a polymer containing sulphonium salt units and then oxidising the sulphonium moieties to sulphoxonium moieties.

Compounds of the invention in which the sulphoxonium group is in the polymer backbone may be prepared by oxidising the corresponding known sulphonium compound. The sulphonium compound may be prepared in known manner from poly (phenylene sulphide) e.g. by reaction with a diphenyliodonium salt. The salt groups may be present at 25 to 100% of the available positions (sulphide groups in the starting polymer), preferably at 100% of the positions.

The compounds of the invention are useful as cationic photoinitiators for cationically polymerizable materials. They may be used in amounts of 0.1 to 30.0, preferably 0.5 to 10 parts by weight per 100 parts by weight of polymerizable material.

They are better cationic photoinitiators than the corresponding sulphonium salts.

A wide range of cationically polymerizable materials may be photopolymerized using compounds of the present invention e.g. those described in US Patent No. 4339567 including epoxides, vinyl compounds, aminoplasts and phenoplasts. Preferred compounds are aromaric glycidyl ethers, especially aromatic diglycidyl ethers.

The sulphoxonium compounds may be used alone or together with a sensitiser to increase the speed of curing. Suitable sensitisers include dyes or aromatic polycyclic compounds such as anthracene, 9-methylanthracene, rubrene, perylene, acenaphthene, phenanthrene, fluoroanthrene and pyrene. Other suitable sensitisers are disclosed in US Patent No. 4069054.

The compounds of the invention liberate less volatile or leachable material into cured films, have higher compatibility with epoxide resins, have less salt aggregation and present lower toxicological hazards.

The invention is illustrated by the following Examples.

Example 1

Polymer related to poly(α-methyl-styrene):

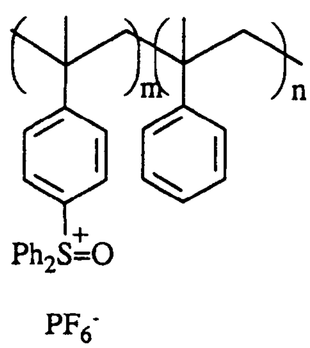

Phenyl sulphoxide (7.26g, 0.036mol) and poly(α-methyl-styrene) (7.14g, 0.072mol, M.W.81000) are dissolved in dichloromethane (90ml) and cooled with stirring in an ice-water bath. Trifluoroacetic anhydride (5.16g, 0.072mol) followed by trifluoromethanesulphonic acid (3.24g, 0.036mol) are added dropwise. After stirring for 30 minutes the mixture is allowed to warm to room temperature over 18 hours. After this time the reaction mixture is added slowly to ether. The ether is decanted and the residue taken up in methanol. The methanolic solution is added dropwise to a solution of potassium hexafluorophosphate (10g) in water. Methanol was removed under reduced pressure. The resulting solid was collected and washed with water, and then with ether to give a solid (12.4g).

A solution of this salt (1.0g) and p-toluenensulphonyl chloride (2.38g, 0.0125mol) in dichloromethane (40ml) is stirred with cooling in an ice-water bath. A solution of sodium peroxide (2.42g, 0.031mol) in water (30ml) is added dropwise over 25 minutes. After a further 1.5 hours stirring is stopped and the two layers allowed to separate. The lower, organic phase is collected, dried and concentrated under reduced pressure. The resulting brown residue is washed with ether to give polymeric sulphoxonium salt (0.81 g) as a syrup. A small quantity is purified as follows: the salt is dissolved in acetone/water and hexafluorophosphonic acid added to

pH4-5. Water is added and the acetone removed in vacuo. The resulting material is collected, washed with water and ether to give the polymeric sulphoxonium salt as a brown solid. mp 79-84°C

NMR($d_6$-acetone):δ 2.1(s) 7.9-8.05(m), 8.2(m)

IR(KBr disk): 1570, 1450, 1080, 845, 750, 730, 700, 680 $cm^{-1}$

Example 2

(a) Fluoro-substituted sulphonium salt intermediate.

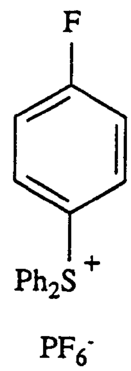

Phenylsulphoxide (10g, 49.5mmol) is dissolved in fluorobenzene (50ml) and cooled to 0°C with stirring. Trifluoroacetic anhydride (20.8g, 14ml, 99mmol) followed by trifluoromethanesulphonic acid (4.4ml, 49.5mmol) are added dropwise. The mixture is further stirred for 18 hours, warming to room temperature. After this time the excess of fluorobenzene is removed under reduced pressure and the residue taken up in aqueous methanol. This solution is added dropwise to an excess of aqueous potassium hexafluorophosphate. The product is extracted three times into dichloromethane and the resulting organic phase dried. Solvent is removed under reduced pressure to give the fluoro-substituted sulphonium salt (10.95g, 52%) as an oil solidifying on standing. mp 102- 105°C.

IR (KBr disk): 1590, 1500, 1480, 1450, 1250, 1165, 840, 750, 685 $cm^{-1}$

NMR($d_6$-acetone):δ 7.65(m,2H), 7.9(m, 10H), 8.05(m,2H)

(b) Reaction of fluoro-salt and poly(4-vinylphenol) to give,

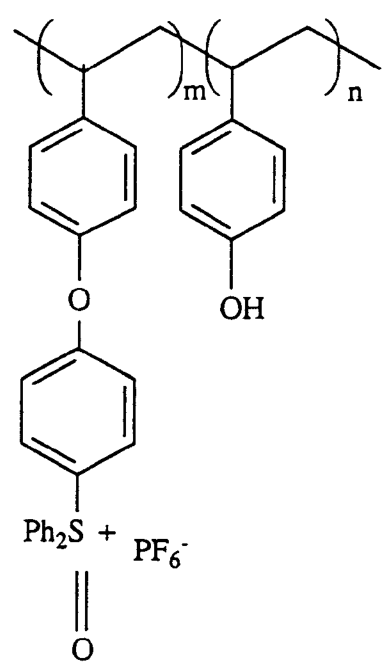

Diphenyl(fluorophenyl)sulphonium hexafluorophosphate (3.0g, 0.007mol) poly(4-vinylphenol) (1.68g, 0.014mol-OH) and potassium carbonate (2.88g, 0.021mol) are stirred at 80-100°C in dimethyl sulphoxide

(90ml) for 18 hours. After this time the reaction mixture is cooled and added dropwise to water. The resulting solid is collected, washed with ether and dried.

A portion (1g) of the product is dissolved with p-toluenesulphonyl chloride ( 1.75g, 0.009mol) in dichloromethane (40ml). The mixture is stirred and cooled in an ice-water bath. A solution of sodium peroxide (1.80g, 0.023mol) in water (30ml) is added dropwise over 20 minutes. After a further 30 minutes stirring is stopped and the two layers allowed to separate. The layers are diluted with water and dichloromethane and the mixture acidified with 2M hydrochloric acid. The lower, organic phase is collected, dried and concentrated under reduced pressure. The resulting solid is collected and washed with ether to give the polymeric sulphoxonium salt (0.47g) as an orange solid, with identical spectra to the product of Example 3.

Example 3

Polymeric sulphoxonium salt from chloro-substituted sulphoxonium salt. Product identical to Example 2.

A stirred mixture of poly(4-vinylphenol) (0.28g, 0.0023mol), diphenyl(chlorophenyl)sulphoxonium hexafluorophosphate (1.0g, 0.0023mol), and potassium carbonate (0.96g, 0.007mol) in dimethyl sulphoxide (30ml) are heated with stirring at 100°C for 18 hours. After this time the reaction mixture is cooled and poured into aqueous potassium hexafluorophosphate. The resulting precipitate is collected and washed with ether. The product is purified by dissolution in acetone/water, addition of aqueous hexafluorophosphoric acid (to pH 4-5) and adding to an excess of water. The resulting off-white solid is collected, washed and dried in vacuo to give the polymeric sulphoxonium salt (1.11 g). mp 185-201°C.

IR(KBr disk): 1590, 1510, 1490, 1085, 850, 760, 730 $cm^{-1}$

NMR($d_6$-acetone) : δ 2.4 (broad), 6.8-7.6(broad), 7.9-8.1(broad)

Example 4

synthesis of:

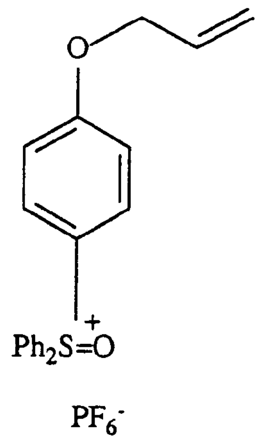

from

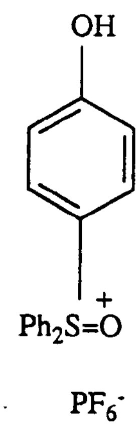

Diphenyl(hydroxyphenyl)sulphoxonium hexafluorophosphate (1.0g, 0.0023mol) allyl bromide (3.0ml) and potassium carbonate (1.5g) an dissolved with stirring in acetone (50ml). The reaction mixture is heated to 65°C for 18 hours. After this time the reaction mixture is filtered and concentrated under reduced pressure. Ether is added and the mixture evaporated in vacuo to give the product (0.21g, 19%) as a yellow oil.

NMR($d_6$-acetone) : δ 4.85(m,2H), 5.4(ddm, 2H), 6.1(m, 1H), 7.5(d,2H), 8.05(m,6H), 8.1 (m,6H).

IR(liq. film) : 1600, 1450, 1280, 1090, 850, 760, 740, 690 $cm^{-1}$

Example 5

synthesis of: 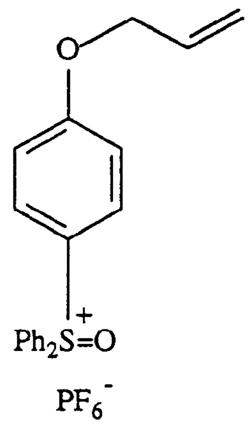 from 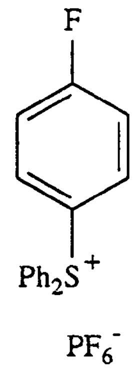

Diphenyl(fluorophenyl)sulphonium hexafluorophosphate (6.0g, 0.014mol), allyl alcohol (1.8ml, 0.03mol) and potassium carbonate (5.76g, 0.04mol) are heated at 80-100°C in dimethyl sulphoxide (180ml) for 18 hours. After this time the reaction mixture is cooled and poured into water. The product is extracted into dichloromethane, which is dried and concentrated under reduced pressure. The resulting product is washed with ether to yield a brown oil (9.9g) contaminated with dimethyl sulphoxide.

A portion of this oil (1g) is oxidised as described in Example 1 to give the allyl substituted sulphoxonium salt (0.24g, 37%) as a yellow wax with spectra identical to those of Example 4.

Example 6

synthesis of: 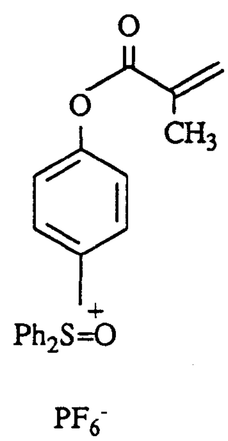 from 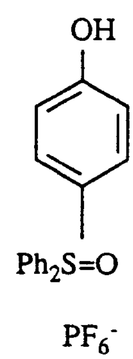

Diphenyl(hydroxyphenyl)sulphoxonium hexafluorophosphate (0.4g, 0.91 mmol) and dimethylaminopyridine (0.25g, 2.0mmol) are stirred at room temperature in dichloromethane (20ml). Methacryloyl chloride (0.1g, 1.0mol) is added slowly. After further stirring for 18 hours the reaction mixture is poured into ether. The resulting pink-coloured material is cooled, washed in water and then ether to give the methacrylate (0.23g, 51 %).
NMR($d_6$-acetone):$\delta$ 2.05 (broad s,3H), 6.0(m,1H), 6.4(m,1H), 7.85(m,2H),8.0(m,4H), 8.2(m,8H).
IR(film) : 1745, 1650, 1580, 1450, 1220, 1085, 850, 760, 730, 680 $cm^{-1}$

Example 7

Adduct of phenolic novolak with sulphoxonium salt:

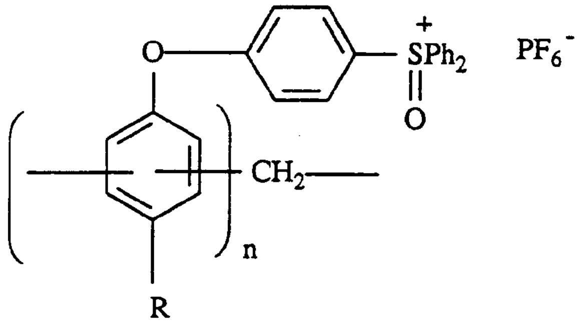

R = 25% $Bu^t$

75% H

Example 3 is repeated but using the polymer Alnoval PN430 instead of poly(4-vinylphenol). The resulting product had mp. 169-176°C.
NMR($d_6$-acetone) : δ 7.9-8.2(m), 3.8(m) v. broad peaks
IR(KBr disk): 1585, 1490, 1250, 1085, 850, 755, 735, 710, 685 $cm^{-1}$

Example 8

Adduct of 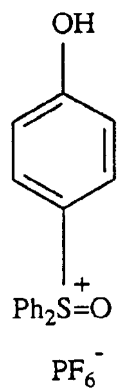 and ARALDITE 6150 (bisphenol A diglycidyl ether advanced by bisphenol A)

Diphenyl(hydroxyphenyl)sulphoxonium hexafluorophosphate (0.5g, 1.1mmol) and ARALDITE 6150 (1.05g, 1.7mmol in epoxide groups) are ground together and heated at 150°C for 2 hours. The mixture is allowed to cool and is dissolved in acetone and water. Hexafluorophosphoric acid (60%) is added until the pH is about 4 and the solution is added to water. The acetone is removed under reduced pressure and the suspension is filtered. The solid product is washed with water and dried in vacuo. Yield 0.61g, mp. 102-108°C.
NMR($d_6$-acetone) : δ 1.60(s,), 3.00(m), 3.90-4.20(m), 6.85(d), 7. 16(d), 7.52(d), 7.99(m), 8. 16(m) broad peaks
IR(KBr disk) : 3500(br), 1610, 1590, 1510, 1250, 1180, 1085, 1040, 830, 760, 730, 680 $cm^{-1}$

Example 9

Adduct of 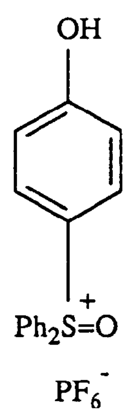 and glycidyl methacrylate

Diphenyl(hydroxyphenyl)sulphoxonium hexafluorophosphate (1.0g, 2.3mmol) and glycidyl methacrylate

(1.0g, 7.0mmol) are heated at 150°C in the presence of 2,6-di-t-butyl-p-cresol (0.05g) for 2½ hours; giving a mixture of adduct and polymeric material.
NMR($d_6$-acetone) :δ 1.05(m), 1.9(m), 3.4-4.6(m), 5.6(m), 6.05(m), 7.5(m), 8.0(m), 8. 1 (m).
IR(film) : 3300, 1720, 1590, 1450, 1180, 1075, 845, 760, 735, 720 $cm^{-1}$

Example 10

synthesis of:

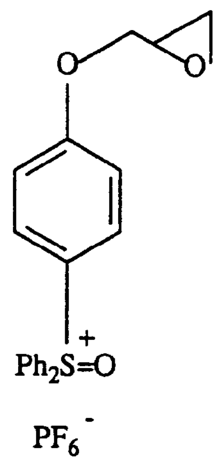

Diphenyl(fluorophenyl)sulphonium hexafluorophosphate (3.0g, 7mmol), glycidol ( 1.2ml, 14mmol) and potassium carbonate (2.88g, 21mmol) are heated at 100°C in dimethyl sulphoxide (100ml) for 18 hours. The mixture is allowed to cool and is poured into water. The product is extracted into dichloromethane which is dried and concentrated under reduced pressure. The resulting product is washed with diethyl ether to yield a brown oil (3.1g). A portion of this oil (1g) is dissolved with p-toluenesulphonyl chloride (2.38g, 12.2mmol) in methanol (40ml). The mixture is stirred and cooled in an ice-water bath. A solution of sodium peroxide (2.42g, 31 mmol) in water (30ml) is added dropwise over 20 minutes. After a further 30 minutes stirring is stopped and the reaction mixture is filtered washing with methanol. The filtrate is concentrated under reduced pressure. The residue is washed with IM aqueous sodium carbonate solution, water and finally diethyl ether. The product is dried in vacuo at 40°C to give a white wax (0.04g).
NMR ($d_6$-acetone) : δ 2.40 (m,2H), 3.61 (,1H), 4. 10(m,2H), 7.62(d,2H), 7.98(m,6H), 8. 13 (m,6H).
IR(squash) : 1595, 1500, 1450, 1150, 1140, 1085, 940, 850, 760, 735, 690 $cm^{-1}$

Example 11

Preparation of the Sulphonium Salt from Poly(phenylene sulphide)

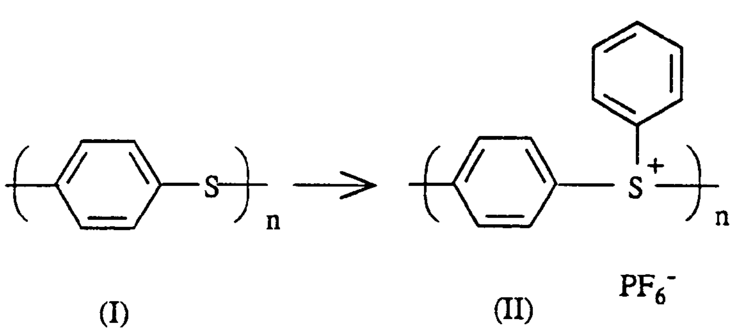

(I) (II)

Poly(phenylene sulphide) (I) (1.0g, 9.26 mmole of sulphide), diphenyliodonium hexafluorophosphate (4.3g, 10mmole) and anhydrous copper (II) acetate (0.08g, 0.44mmol) are stirred as a melt at 140-160°C for 4 hours. The colourless liquid is decanted. The remaining oil is washed with diethyl ether (2 x 50 ml), water (2 x 50 ml) and dissolved in acetone (25 ml). The solution is dropped into diethyl ether (200 ml) and a brown precipitate forms which is collected and dried to give the sulphonium salt (II) (0.65g, 13%) m.p. 234-6°C (decomp).

N.M.R. ($d_6$-acetone) δ 7.90 (br.,m.).

I.R. (KBr disk) 1580, 1450, 1410, 1360, 1050, 990, 840, 760, 680 $cm^{-1}$.

The Preparation of the Sulphoxonium Salt from the Sulphonium Salt

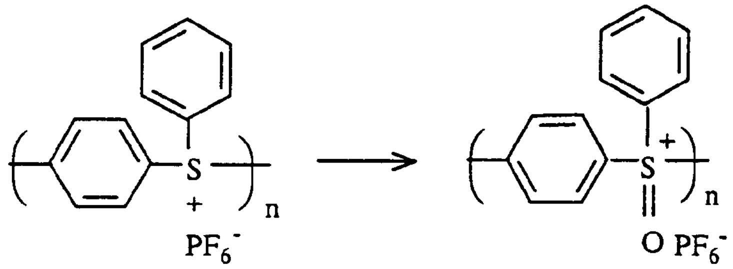

(II) (III)

The sulphonium salt (II) (0.5g, 0.936mmole of sulphonium groups) is dissolved in methanol (50ml) and the solution is cooled to 0°C. p-Toluenesulphonyl chloride (1.2g, 5.88mmole) and sodium peroxide (1.1g, 14mmole) in water (15ml) are added. The mixture is stirred for 1 hour and is then concentrated to remove the methanol. The product is extracted into dichloromethane (3 x 50ml), the combined organic layers are dried ($MgSO_4$) and concentrated. The crude product is purified by dissolving it acetone (200ml) and adding the solution to a solution of potassium hexafluorophosphate (5g) in water (100 ml). The mixture is cooled and the precipitate is collected and dried to give the sulphoxonium salt (III) (0.1g, 19%) m.p.>300°C.

N.M.R. ($d_6$-acetone): $\delta$ 8.20 (br) and 7.80 (br).

I.R. (KBr disk): 1580, 1470, 1440, 1320, 1160, 1070, 1010, 840, 750, 720, 680 $cm^{-1}$.

Examples 12-18

Compositions comprising 2,2-bis(p-glycidyloxyphenyl)propane (100 parts by weight) a sulphoxonium salt as indicated in the Table below (5 parts by weight) with 2 parts by weight of 9-methyl-anthracene as sensitiser, are applied as a film 24 microns thick on tinplate. They are then exposed in two ways to produce a tack free coating. The first is simple exposure to a metal halide lamp (5000 w) at a distance of 75 cm and the time taken to become tack-free is recorded. The second is to expose the film to radiation from a medium pressure mercury arc lamp (80W per cm) at a distance of 8 cm on a Primarc type apparatus running at 21.3 m/min. The number of passes needed for a tack-free coating is recorded.

| Example | Salt from Example | Time | No. of Passes |
|---|---|---|---|
| 12 | 1 | 0.5 min | 3 |
| 13 | 3 | 1.5 min | 14 |
| 14 | 5 | 1.5 min | 7 |
| 15 | 7 | 1.0 min | 7 |
| 16 | 8* | - | 19 |
| 17 | 9 | 2.5 min | 12 |
| 18 | 10 | - | 16 |

* used at 10 parts by weight

Examples 19-20

Compositions comprising 2,2-bis(p-glycidyloxyphenyl)propane (100 parts by weight) and the polymeric sulphoxonium salt III from Example 11 (3 parts by weight) with or without 9-methylanthracene as sensitiser (1 part by weight) are applied as a film, 24 microns thick on copper clad laminate. They are exposed to a metal halide lamp (5000 W) at a distance of 75 cm and the time taken to become tack-free is recorded.

| Example | Compound | Time |
|---|---|---|
| 19 | Salt III with so sensitiser | 160 seconds |
| 20 | Salt III with sensitiser | 65 seconds |

## Claims

1. An aryl sulphoxonium salt which either contains a polymerizable group in the molecule or is part of a polymeric compound.

2. A sulphoxonium salt as claimed in claim 1 which is a triaryl sulphoxonium salt where one of the aryl groups is pendant on a polymeric chain.

3. A sulphoxonium salt as claimed in claim 2 in which the polymer is one containing phenyl groups or is an epoxy resin.

4. A sulphoxonium salt as claimed in claim 2 in which the sulphoxonium salt is present on the chain at from 25 to 75% of the possible sites, or is the terminating group of an oligomer.

5. A sulphoxonium salt as claimed in claim 1 which is a triaryl sulphoxonium salt where one of the aryl groups is substituted by a polymerisable group.

6. A sulphoxonium salt as claimed in claim 5 in which the polymerizable group is one containing ethylenic unsaturation or is a 1,2-epoxide group.

7. A sulphoxonium salt as claimed in claim 1 which has the general formula I

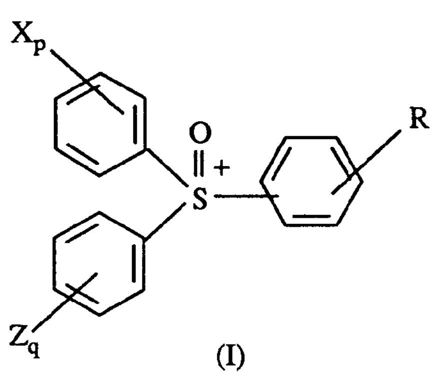

(I)

where R contains a (meth)acrylate, a vinyl ether, an allyl ether or an epoxy group, X and Z, which may be the same or different, represent monovalent substituents and p and q, which may be the same or different are either zero or integers below 6.

8. A sulphoxonium salt as claimed in claim 1 in which the sulphoxonium group is in a polymer backbone.

9. A sulphoxonium salt as claimed in claim 1 in which the anion is a halogen, trifluoromethanesulphonate, or an ion of the formula (II)

$$MY^{-}_{m} \quad (II)$$

where M is an atom of a metal or metalloid, Y is a halogen atom and m is 4, 5 or 6 and is one more than the valency of M.

**10.** A process for preparing a sulphoxonium salt as claimed in claim 1 which comprises either reacting an aryl sulphoxonium salt containing a reactive group with a compound which will introduce the desired polymerizable group, or by reacting an aryl sulphonium salt containing a reactive group with a compound which will introduce the desired polymerizable group and then oxidising the sulphonium moieties to sulphoxonium moieties.

**11.** A process for preparing a sulphoxonium salt as claimed in claim 1 which comprises either reacting a polymer containing reactive groups with a sulphonium or sulphoxonium salt containing a group reactive with the reactive group on the polymer and if necessary convening the sulphonium salt into the corresponding sulphoxonium salt, or by reacting an aromatic polymer with a diaryl sulphoxide to produce a polymer containing sulphonium salt moieties and then oxidising the sulphonium moieties to sulphoxonium moieties.

**12.** A process for preparing a sulphoxonium salt as claimed in claim 1 in which a sulphonium salt having sulphonium groups in a polymer backbone is oxidised to the corresponding sulphoxonium salt.

**13.** The use of sulphoxonium salts as claimed in any one of claims 1 to 11 as cationic photoinitiators for cationically polymerizable materials.

**14.** The use as claimed in claim 13 in which 0.1 to 30.0 parts by weight of sulphoxonium salt are present per 100 parts by weight of polymerisable material.

European Patent Office

# EUROPEAN SEARCH REPORT

Application Number

EP 92 30 3173

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-0 035 969 (CIBA-GEIGY) *claims* * page 38, paragraph 2 * & US-A-4 339 567 | 1-14 | C08F2/50 G03F7/029 |
| A | EP-A-0 022 081 (CIBA-GEIGY) *claims* | 1-14 | |
| A | EP-A-0 380 010 (BASF) | 1-14 | |

TECHNICAL FIELDS SEARCHED (Int. Cl.5 )

C08F
G03F

The present search report has been drawn up for all claims

| Place of search | Date of completion of the search | Examiner |
|---|---|---|
| THE HAGUE | 10 JULY 1992 | ANDRIOLLO G. R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)